Europäisches Patentamt

European Patent Office

Office européen des brevets

(11) Publication number: 0 386 848 A1

# EUROPEAN PATENT APPLICATION

(21) Application number: 90200523.0

(22) Date of filing: 05.03.90

(51) Int. Cl.5: C12N 1/14, C12P 1/00, C12P 41/00, //(C12N1/14, C12R1:645)

The applicant has filed a statement in accordance with Rule 28 (4) EPC (issue of a sample only to an expert). Accession number(s) of the deposit(s): CBS 547.88.

(30) Priority: 09.03.89 EP 89200603

(43) Date of publication of application:
12.09.90 Bulletin 90/37

(84) Designated Contracting States:
AT BE CH DE DK ES FR GB GR IT LI LU NL SE

(71) Applicant: GIST-BROCADES N.V.
Wateringseweg 1 P.O. Box 1
NL-2600 MA Delft(NL)

(72) Inventor: De Smet, Marie José
Groenhovenweg 383
NL-2803 EK Gouda(NL)

(74) Representative: Matulewicz, Emil Rudolf
Antonius, Dr. et al
Gist-Brocades NV Patents and Trademarks
Department Wateringseweg 1 P.O. Box 1
NL-2600 MA Delft(NL)

(54) Novel Exophiala species.

(57) A novel Exophiala species viz. Exophiala wilhansii is disclosed. This species is advantageously applied in isomerization and oxidation reactions, and in stereoselective enrichment of one isomer of certain (R,S)-compounds by the conversion of the other isomer.

EP 0 386 848 A1

## Novel Exophiala species

The invention relates to a novel black yeast-like fungus: Exophiala wilhansii and its use in certain stereospecific syntheses.

The morphology of this novel black yeast-like fungus was determined by the Centraal Bureau voor Schimmelcultures, Baarn, Holland. It was found that the black yeast-like fungus belongs to the group of Ascomycetous black yeasts. More specifically, the novel species was distinguished as belonging to the genus Exophiala. The characteristics of the novel species correspond to those of the Exophiala genus as described by G.S. de Hoog et al. in "The expanding realm of yeast-like fungi, Proceedings of an international symposium on the perspectives of taxonomy, ecology and phylogeny of yeasts and yeast-like fungi", Amersfoort, The Netherlands, 3-7 August 1987, Studies in Mycology no. 30 (1987), p. 187-199, Ed. G.S. de Hoog et al., publ. Elsevier Science Publishers, Amsterdam and K. Nishimura and M. Miyaji in Mycopathologia 81, p. 135-144 (1983). Further determination showed that the novel species was closely related to Exophiala dermatitidis (Kano) de Hoog (see De Hoog et al. vide supra, page 190). The main difference is the positive nitrogen assimilation of the novel species. Another difference is the occurrence of sclerotial bodies which are absent in the Exophiala dermatitidis. An example of the novel species Exophiala wilhansii has been desposited with the Centraal Bureau voor Schimmelcultures (CBS), Oosterstraat 1, Baarn, The Netherlands as CBS 547.88 on September 6, 1988.

Mutants and variants can be obtained from the novel species Exophiala wilhansii (CBS 547.88) using procedures known per se. For example, mutants can be obtained using 1,2,7,8 diepoxyoctane (DEO) (Hynes M.J. (1979) Genetics 91: 381-392); ethylmethanesulfphonate (EMS) (Fink G.R. (1970) Methods enzymol. 17 (A): 59-78); N-methyl-N'-nitro-H-nitrosoguanidine (NTG) (Hagino H. and Nakayama K. (1973) Agr. Biol. Chem. 37 (9): 2007-2011) or a standard ultraviolet irradiation procedure.

A biological examination was carried out as described by K. Nishimura and M. Miyaji in Mycopathologia 77, p. 173-181 (1982). The results of this examination are given in Table 1. In order to show the differences with Exophiala dermatitidis the examination of this species is also given in Table 1.

Table 1

| Assimulation of carbon compounds | Exophiala dermatitidis (CBS 207.35) | Exophiala wilhansii (CBS 547.88) |
|---|---|---|
| glucose | + | + |
| fructose | + | + |
| xylose | + | + |
| galactose | + | + |
| mannose | + | + |
| rhamnose | + | + |
| arabinose | + | + |
| mannitol | ± | ± |
| maltose | + | + |
| saccharose | + | + |
| lactose | - | - |
| melibiose | - | - |
| raffinose | - | - |
| hydrolyse of starch | - | - |
| splitting of arbutin | ± | ± |
| assimulation of KNO₃ | - | + |
| hydrolysis of skin milk | - | - |
| hydrolysis of gelatin | - | - |
| hydrolysis of urea | + | + |

The species can be kept in frozen stocks. The species is advantageously applied in isomerization and oxidation reactions. The species shows an isomerization activity for substituted α-propionic acids such as fluazifop, diclofop, naproxen and ibuprofen. The novel species shows oxidation activity for substituted alkanes, preferably 2-substituted propanes. The stereospecific oxidation products are the corresponding alcohols or acids. If desired, the alcohol can be oxidized further to the acid with the aid of the Exophiala species. An example of such oxidation is the conversion of 2-(6-methoxy-2-naphthyl)propane into (R)-2-(6-methoxy-2-naphthyl)propanol and (S)-2-(6-methoxy-2-naphthyl)propanoic acid.

Moreover it was found that (R,S)-compounds could be enriched in the (R)-isomer by the conversion of the (S)-component. For example, (R,S)-naproxen amide and (R,S)-phenyl glycine could be enriched in the (R)-isomer by the novel Exophiala species.

Amongst other things the isomerization capacity characterizes the novel species which can be deduced from the fact that other Exophiala species do not show isomerization activity for e.g. naproxen (Exophiala jeanselmei var. lec CBS 123.33, Exophiala jeanselmei CBS 537.76 and Exophiala mansonii CBS 101.76 were tested).

## EXPERIMENTAL

### Brief description of the Figure

The Figure shows growth curves for Exophiala wilhansii (CBS 547.88) during incubation with different carbon sources. The experiments were performed four, three, and one times, for glycerol (0.5%), glucose (0.5%), and Tween-80 (TM) (1.25%), respectively. The curves present typical cases for each set of experiments.

| Brief description of the media used | |
|---|---|
| BHI-medium | |
| Brain heart infusion | 37 g/l |
| PDA-medium | |
| Potato dextrose agar | 39 g/l |
| YEPD-medium | |
| Bactopeptone (TM) | 20 g/l |
| Yeast extract | 10 g/l |
| Glucose | 20 g/l |
| pH adjusted to 7.0 | |
| Minimal salts medium (PSIII) pH 7.0 | |
| $KH_2PO_4$ | 2.1 g/l |
| $(NH_4)2HPO_4$ | 1.0 g/l |
| $(NH_4)_2HSO_4$ | 0.9 g/l |
| KCl | 0.2 g/l |
| $CaSO_4.2H_2O$ | 0.005 g/l |
| $MgSO_4.7H_2O$ | 0.2 g/l |
| $(NH_2)SO_4.FeSO_4.6H_2O$ | 2.5 mg/l |
| $ZnSO_4.7H_2O$ | 0.5 mg/l |
| $MnCl_2.4H_2O$ | 0.3 mg/l |
| $CuSO_4.5H_2O$ | 0.15 mg/l |
| $CoCl_2.6H_2O$ | 0.15 mg/l |
| $H_3BO_3$ | 0.05 mg/l |
| $Na_2MoO_4.2H_2O$ | 0.055 mg/l |
| KI | 0.1 mg/l |
| pH adjusted to 6.8 All media are sterilized for 20 minutes at 120°C. | |

## Isolation of Exophiala wilhansii (CBS 547.88)

About 1 gram soil sample taken from Shellhaven Refinery (Pig bay sludge farm, UK) was suspended in 10 ml physiological salt solution supplemented with yeast extract at 30°C for 3 hours the mixture was diluted 20-fold in BHI-medium supplemented with penicillin-G (20 μg/ml), globenicol (40 μg/ml), and streptomycine (15 μg/ml). The dilution was incubated at 30°C for 4 hours on a shaker at 300 rpm.

Subsequently samples were diluted 6-fold in minimal salts medium (PSIII) supplemented with 2-(6-methoxy-2-naphthyl)propane (4 mg/ml).

The cultures were incubated at 30°C for 5 days on a shaker at 200 rpm. Samples were taken periodically and plated onto the corresponding medium. Single colonies were purified and screened for the ability to oxidize 2-(6-methoxy-2-naphthyl)propane.

A fungus was isolated which was capable of oxidizing 2-(6-methoxy-2-naphthyl)propane. This microorganism could not use this substrate as sole source of carbon and energy.

## Growth characteristics of Exophiala wilhansii

i. Growth conditions

4

Exophiala wilhansii (CBS 547.88) was kept in frozen stocks and inoculated for use into a minimal salts medium (PSIII) supplemented with yeast extract (0.01 w/v%) and glycerol (0.5 w/v%) and grown at 30° C for 48 to 68 hours.

The culture was diluted 20 to 100-fold in fresh medium and grown at 30° C or 37° C for 20 to 24 hours. Subsequently the culture was diluted 20 to 100-fold in fresh minimal salts medium (PSIII) supplemented with yeast extract (0.01 w/v%) and glycerol (0.5 w/v%), glucose (0.5-1.0 w/v%) or Tween-80 (TM) (1.25 w/v%).

The cultures were grown at 30° C or 37° C for the time indicated. Cell densities and cell viabilities were determined as described below.

All experiments were conducted in 500 ml baffled shake flasks containing 100 ml of medium, and which were shaken at 200 rpm.

ii. Determination of the cell density and cell viability

Cell densities (mg of cell dry mass/ml) were obtained by determining the cell dry weight. The cells from the culture sample (5 ml) were collected by centrifugation and washed once in physiological salt solution prior drying under defined conditions. The obtained dry weights were corrected for the weight of the remaining salts.

For determination of the cell viability cultures were serially diluted in physiological salt solution. Dilutions were plated on BHI-medium and the plates were incubated at 30° C for two days.

Exophiala wilhansii grows well on/in rich media at 30° C and 37° C like YEPD, BHI, and PDA. Dry weights up to 12 mg cell dry mass per ml were reached in such cultures (results not shown).

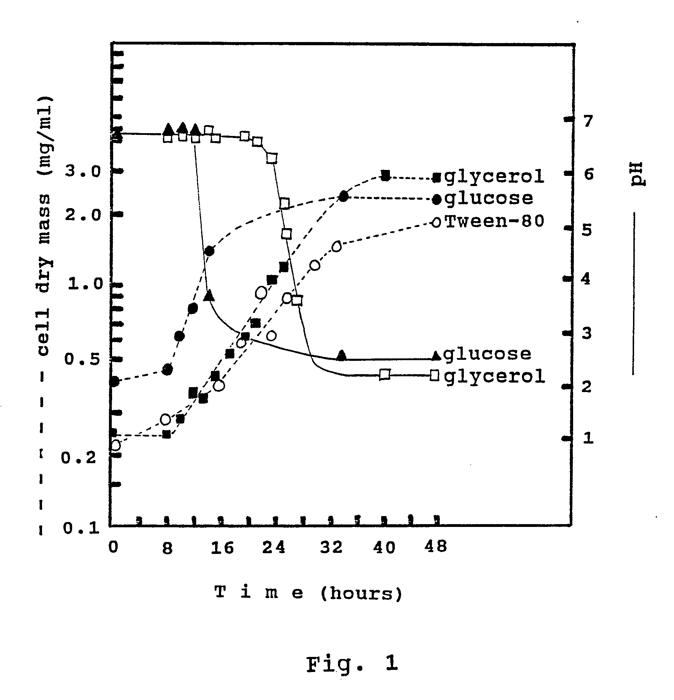

Figure 1 shows typical growth curves for this microorganism with different carbon sources at 30° C incubation temperature. When glycerol (0.5%) was the carbon source the growth rate in the exponential phase was found to vary from 0.07 to 0.10 hr$^{-1}$ (doubling time: 9.5 to 7 hours). In a culture with glucose (0.5 or 1.0%) the growth rate in the exponential phase was 0.13 to 0.17 hr$^{-1}$ (doubling time: 5.5 to 4 hours). As indicated in Figure 1 the pH decreased dramatically during the late exponential growth phase (from 6.6 to 3.5). During the stationary growth phase the pH remained constant at 2.2. The microorganism was capable of using Tween-80 (TM) as sole source of carbon and energy.

All growth experiments were repeated at 37° C. It was found that there were no significant differences in growth rates at 37° C and 30° C. The effect of the pH of the growth medium was investigated in more detail. Minimal salts media buffered at pH 4.0, pH 7.0, and pH 9.0 were prepared by incorporating 0.1 M phosphate citrate buffer pH 4.0 or 7.0, or 0.1 M carbonate hydrogen carbonate buffer pH 9.0 into PSIII-medium, respectively.

The media were supplemented with glycerol (0.5%) and yeast extract (0.01%). Growth of Exophiala wilhansii at pH 4.0 was similar to the growth at pH 7.0, the latter being similar to the growth pattern in non-buffered PSIII. No growth was observed at pH 9.0.

During growth of Exophiala wilhansii in the media mentioned above the cells which were white during early exponential phase slowly turned black through subsequent growth phases until stationary phase was reached. Cell viability remained >90% (expressed as a percentage of the total number of cells expected on basis of the dry weight of the cells).

Example 1

The Exophiala wilhansii (CBS 547.88) was inoculated for use into a minimal salts medium (PSIII) supplemented with yeast extract (0.01 w/v%) and glycerol (0.5 w/v%) and grown at 30° C for 48 hours in a 500 ml baffled flask containing 100 ml medium with shaking.

The culture was diluted 20-fold in fresh medium and grown at 30° C for 24 hours. The cell mass was collected by centrifugation and resuspended in the same medium without glycerol. The cell concentration of the suspension was 10-15 mg cell dry mass per ml.

Two ml soyoil or 2 ml 7.5% Tween-80 (TM) or 2 ml medium containing 5 mg substrate (2-(6-methoxy-2-naphthyl)propane, 2-(6-methoxy-2-naphthyl)propanol or 2-(6-methoxy-2-naphthyl)propanoic acid) was added to 10 ml suspensions. The mixtures were shaken in 125 ml baffled flasks at 30° C for the time indicated.

The suspensions were acidified to pH 2.0 with 85% phosphoric acid and then extracted with dichloromethane.

5

The quantities of acid and alcohol recovered were determined by HPLC. The enantiomeric ratios of the alcohol and the acid were determined by chiral HPLC, directly and after derivatization, respectively. The presence of 2-(6-methoxy-2-naphthyl)propanoic acid was confirmed using UV spectroscopy.

Quantification of 2-(6-methoxy-2-naphthyl)propane, 2-(6-methoxy-2-naphthyl)propanol, and 2-(6-methoxy-2-naphthyl)propanoic acid (naproxen).

Two ml of extract was concentrated by evaporation under nitrogen at 60° C. The residue was dissolved in 2 ml acetonitrile/water (2:1, v/v) and analyzed with HPLC using a Chrompack Polygosyl 60 D 10 CN column (250 mm, diameter 4.6 mm) eluted with acetonitrile/0.03 M NaH₂PO₄ (36:64, v/v) pH 5.0. compounds were detected spectrophotometrically at 254 nm.

Determination of the enantiomeric purity of 2-(6-methoxy-2-naphthyl)propanoic acid (naproxen), 2-(6 methoxy-2-naphthyl)propanamide (naproxenamide), and 2-(4-isobutyl-1-phenyl)propanoic acid (ibuprofen).

Eight to twelve ml of extract was concentrated by evaporation under nitrogen at 60° C. The dried residue was dissolved in 2 ml dichloromethane and allowed to react for 10 min at 60° C with 200 μl of a solution of 2-bromo-1-methyl-pyridiniumiodide in dimethylformamide (50 mg/ml) and 200 μl of a solution of 1-naphthalene methylamine in dichloromethane (100 mg/ml). The reaction mixture was dried under nitrogen at 60° C. The residue was dissolved in 2 ml isooctane/dichloromethane (2:1, v/v) and extracted after addition of 2 ml 1 N HCl. The organic layer was dried and analyzed with HPLC using a Baker, aminopropyl covalent DNBPG column (250 nm, diameter 4.6 mm) and eluted with isooctane/chloroform/methanol (90:7:3, v/v/v). Compounds were detected spectrophotometrically at 280 nm.

Determination of the enantiomeric purity of 2-(6-methoxy-2-naphthyl)propanol.

Extracts were analyzed directly with HPLC using a chiral 2 column (Macherey-Nagel, Düren; 250 nm, diameter 3.6 mm) eluted with isooctane/isopropanol/methanol (97:2:1, v/v/v). Compounds were detected spectrophotometrically at 254 nm.

Results

Table 2

| Conversion of (R/S)-2-(6-methoxy-2-naphthyl)propionic acid into (S)-2-(6-methoxy-2-naphthyl)propionic acid using the Exophiala wilhansii . | | | |
|---|---|---|---|
| detergent/oil present | incubation (hrs) | enantiomeric ratio (R/S) | recovery (%) |
| none | 24 | 32/68 | 74 |
| Tween-80 | 16 | 0/100 | 92 ± 14* |
| Soyoil | 16 | 0/100 | 43 |

*Experiment has been performed in triplicate

6

Table 3

| Conversion of the (R) and the (S)-enantiomer of 2-(6-methoxy-2-naphthyl)-propionic acid using the Exophiala wilhansii . | | | |
|---|---|---|---|
| substrate* | incubation (hrs) | enantiomeric ratio (R/S) | recovery (%) |
| R-enantiomer | 8 | 61/39 | 100 |
| | 16 | 0/100 | 80 |
| S-enantiomer | 8 | 0/100 | 98 |
| | 16 | 0/100 | 98 |

* Substrate was added as a solution in 7.5% Tween-80 (TM) as described above.

Table 4

| Conversion of (R/S)-2-(6-methoxy-2-naphthyl)propanol into (S)-2-(6-methoxy-2-naphthyl)propionic acid using the Exophiala wilhansii . | | | | | |
|---|---|---|---|---|---|
| detergent/oil present | incubation (hrs) | enantiomeric ratio of | | recovery* (%) of | |
| | | alcohol (R/S) | acid (R/S) | alcohol | acid |
| none | 16 | 56/44 | 2/98 | 75 | 25 |
| Tween-80 | 16 | 58/42 | 27/73 | 67 | 19 |
| Soyoil | 16 | 50/50 | 25/75 | 73 | 20 |

*Expressed as the % (mole) of the substrate added.

Table 5

| Conversion of the (R) and the (S)-enantiomer of 2-(6-methoxy-2-naphthyl)-propanol into (S)-2-(6-methoxy-2-naphthyl)-propanoic acid using the Exophiala wilhansii . | | | | | |
|---|---|---|---|---|---|
| Substrate* | incubation (hrs) | enantiomeric ratio of | | recovery** (%) of | |
| | | alcohol (R/S) | acid (R/S) | alcohol | acid |
| R-enantiomer | 8 | 100/0 | 0/100 | 69 | 8 |
| | 16 | 100/0 | 0/100 | 35 | 16 |
| S-enantiomer | 8 | 0/100 | 0/100 | 81 | 11 |
| | 16 | 0/100 | 0/100 | 59 | 28 |

* Substrate was added as a solution in 7.5% Tween-80 as described above.
** Expressed as the % (mole) of the substate added.

Table 6

| Conversion of 2-(6-methoxy-2-naphthyl)propane* into 2-(6-methoxy-2-naphthyl)propanol and 2-(6-methoxy-2-naphthyl)propanoic acid using the Exophiala wilhansii . | | | | |
|---|---|---|---|---|
| incubation (hrs) | enantiomeric ratio of | | recovery (%)** of | |
| | alcohol (R/S) | acid (R/S) | alcohol | acid |
| 8 | 100/0 | 0/100 | 7 | 5 |
| 16 | 90/10 | 0/100 | 12 | 11 |

\* Substrate was added as a solution in 7.5% Tween-80 (TM) as described above.

\*\* Expressed as the % (mole) of the substate added.

This example shows that the Exophiala wilhansii is capable of converting (R)-2-(6-methoxy-2-naphthyl)-propionic acid into (S)-2-(6-methoxy-2-naphthyl)propionic acid with a yield of 100%. Moreover the Exophiala species is capable to stereospecifically oxidize 2-(6-methoxy-2-naphthyl)propane into (R/S)-2-(6-methoxy-2-naphthyl)propanol and finally into (S)-2-(6-methoxy-2-naphthyl)propionic acid .

Although Tween-80 (TM) is not essential for the isomerization process, higher conversion rates are achieved in the presence of this detergent.

Example 2

Effect of cell age on the isomerization of naproxen

Exophiala wilhansii (CBS 547.88) was grown for 24 hours at 30° C in a 125 ml baffled flask containing 25 ml minimal salts medium (PSIII) supplemented with yeast extract (0.01 w/v%) and glucose or glycerol (0.5 w/v%). After this period the culture was diluted 20-fold in the same fresh medium. The culture was incubated at 30° C. The cultures were harvested as soon as the indicated growth phase was reached. The cells were resuspended in minimal salts medium. The cell concentration was 12-20 mg cell dry mass per ml. Two ml 7.5 v/v% Tween-80 (TM) containing 5 mg naproxen was added to 10 ml suspension. The mixtures were shaken at 30° C for the time indicated. Samples were extracted with dichloromethane after acidification to pH 2.0 with 85% phosphoric acid. The quantity and enantiomeric ratio of the naproxen were determined as described in Example 1.

The results of the effect of cell age on the isomerization of naproxen are presented in Table 7.

Table 7

| Effect of cell age on isomerization of naproxen by Exophiala wilhansii | | | |
|---|---|---|---|
| growth phase | incubation (hrs) | enantiomeric ratio (R/S) | recovery (%) |
| mid exponential | 4 | 50/50 | 88 |
| mid exponential | 8 | 45/55 | 85 |
| early stationary (late exponential) | 4 | 50/50 | 93 |
| early stationary (late exponential) | 8 | 10/90 | 80 |
| stationary | 4 | 50/50 | 94 |
| stationary | 8 | 16/84 | 62 |
| All experiments have been performed twice. The deviation never exceeded 10% of the value shown. | | | |

We also examined the effect of the pH of the suspension on the isomerization activity. Cells harvested from early stationary phase cultures were resuspended in:
0.1 M phosphate buffer pH 7.0, 0.1 M citrate phosphate buffer pH 5.0, 0.1 M citrate buffer pH 3.0, or in minimal salts medium adjusted to pH 7.0, pH 5.0 or pH 3.0. No significant differences in isomerization activity were observed.

Example 3

Isomerization of several substituted α-propionic acids

Culturing of Exophiala wilhansii (CBS 547.88) and conditions for incubation of this strain with the substrate were the same as described under Example 2. Cells harvested from early stationary phase cultures were used for all experiments. After the incubation period samples were acidified and extracted with dichloromethane with the exception of the samples containing fluazifop which were extracted with ethyl acetate.
The extracts were analyzed as follows.

Quantification of fluazifop and diclofop acid

The extracts were analyzed by HPLC using a chrompack Cp-spher Sil column (250 nm, diameter 4.6 mm) eluted with isooctane/ethyl acetate/formic acid (875:125:3.5, v/v/v). The same procedure as described before is used.

Determination of the enantiomeric purity of fluazifop acid and diclofop acid.

The extracts (4-10 ml) were concentrated by evaporation under nitrogen. The residue was mixed with 0.5 ml $BF_3$ in methanol (11 v/v%) and incubated at 60°C for 20 minutes. The mixture was dried under nitrogen. The residue was dissolved in 4 ml n-heptane/tetrahydrofuran/isopropanol (1000:8:1, v/v/v) in case of fluazifop and in 2 ml n-hexane in case of diclofop. The solution was shaken with 1 ml 0.1 M phosphate buffer and after extraction the organic layer was analyzed with HPLC using a Et 250/8/4 Nucleosil chiral-2 column (Macherey-Nagel, Düren) eluted with n-heptane/tetrahydrofuran/isopropanol (1000:8:1, v/v/v) for fluazifop and with n-heptane/isopropanol (1000:1, v/v) for diclofop.
The results of the isomerization of several substituted α-propionic acids are presented in Table 8.

Table 8

| substrate | incubation (hrs) | enantiomeric ratio (R/S) | recovery (%) |
|---|---|---|---|
| (R,S)-fluazifop | 6 | 60/40 | 100 |
| | 24 | 95/5 | 100 |
| (R,S)-diclofop | 6 | 59/41 | 61 |
| | 24 | 99/1 | 69 |
| (R,S)-ibuprofen | 4 | 52/48 | 100 |
| | 8 | 66/34 | 100 |
| All experiments have been performed in triplicate. The deviations never exceeded 10% of the value shown. | | | |

Example 4

Stereospecific conversion of (R,S)-naproxenamide

Culturing of Exophiala wilhansii (CBS 547.88) and conditions for incubation of this strain with (R/S)-naproxenamide were the same as described in Example 1. After the incubation period samples were extracted with dichloromethane. The quantity and the enantiomeric purity of the naproxenamide were determined as described in Example 1.

The results of the stereospecific conversion of (R,S)-naproxen amide are given in Table 9.

Table 9

| | Incubation period | |
|---|---|---|
| | 8 hrs | 24 hrs |
| recovery (%) of naproxenamide enantiomeric ratio (R/S) | 88 56/44 | 53 77/23 |
| The experiments have been performed twice. The deviation never exceeded 10% of the value shown. | | |

Example 5

Stereospecific conversion of (R,S)-phenylglycine

Culturing of Exophiala wilhansii (CBS 547.88) and conditions for incubation of this strain with (R,S)-phenylglycine were the same as described in Example 1. Cells harvested from early starionary phase cultures were used for all experiments. After the incubation period samples were centrifuged and the supernatants were analyzed by thin layer chromatography (Chiral plate, Macherey-Nagel, Düren). The plates were eluted with methanol-water-acetonitrile (50:50:200, v/v/v). The compounds were visualized by spraying with a 0.1% ninhydrin spray reagent.

The results are presented in Table 10.

Table 10

| | Incubation period | |
|---|---|---|
| | 8 hrs | 24 hrs |
| recovery of phenylglycine (%)* enantiomeric ratio (R/S) | 70 not determined | 50 100/0 |

\* estimated by comparison with a standard.
The experiments have been performed in triplicate.

## Claims

1. A strain of the species Exophiala wilhansii which is capable of the isomerization of an optically active substituted α-propionic acid or derivative thereof and which is substantially free from other fungal material.

2. A strain of the species Exophiala wilhansii according to claim 1 which is CBS 547.88 or a mutant or variant thereof.

3. A microbiological process which comprises the use of a strain of the species Exophiala wilhansii.

4. A microbiological process which comprises the use of Exophiala wilhansii (CBS 547.88).

5. A microbiological process which comprises the use of a strain of the species Exophiala wilhansii in an oxidation reaction or in an isomerization reaction.

6. A microbiological process which comprises the use of a strain of the species Exophiala wilhansii in the enrichment of an (R,S)-compound in one isomer by the conversion of the other isomer.

7. A process for the isomerisation of an optically active substituted α-propionic acid or derivative thereof or the stereospecific oxidation of a substituted alkane, which comprises incubating, with a sample of the said acid or alkane, cells of a strain of the species Exophiala wilhansii suspended in a medium.

8. A process according to claim 7 for producing an (R)-isomer-rich optically active substituted α-propionic acid or derivative thereof which comprises incubating, with a sample of the (R,S)-isomer of the said acid or derivative, cells of a strain of the species Exophiala wilhansii suspended in a medium and then extracting the (R)-isomer-enriched acid from the medium.

9. A process according to claim 7 or 8 wherein the optically active α-propionic acid or derivative thereof is 2-(6-methoxy-2-naphthyl)propanoic acid, 2-(6-methoxy-2-naphthyl)propanamide; 2(4-isobutyl-1-phenyl)-propanoic acid, fluazifop acid or diclofop acid.

10. A process according to claim 7 for producing an (R)-isomer-rich optically active substituted alcohol and/or an (S)-isomer-rich optically active substituted carboxylic acid which comprises incubating, with a sample of the substituted alkane corresponding to the said alcohol or acid, cells of a strain of the species Exophiala wilhansii suspended in a medium, and then extracting the (R)-isomer-enriched alcohol and/or the (S)-isomer-enriched acid from the said medium.

11. A process according to claim 10 wherein the substituted alkane is a 2-substituted propane.

Fig. 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 274 146 (GIST-BROCADES)<br>* Claims *<br>----- | 1 | C 12 N   1/14<br>C 12 P   1/00<br>C 12 P   41/00   //<br>(C 12 N   1/14<br>C 12 R   1:645) |
| | | | **TECHNICAL FIELDS SEARCHED (Int. Cl.5)**<br><br>C 12 P<br>C 12 R |

The present search report has been drawn up for all claims

| Place of search | Date of completion of the search | Examiner |
|---|---|---|
| THE HAGUE | 21-06-1990 | DELANGHE L.L.M. |